# EUROPÄISCHE PATENTSCHRIFT

(11) **EP 1 658 880 B1**
(45) Veröffentlichungstag und Bekanntmachung des Hinweises auf die Patenterteilung: **30.06.2010**
(21) Anmeldenummer: 05023488.9
(22) Anmeldetag: 27.10.2005
(51) Int. Cl.: A61Q 13/00, C11D 1/08, C11D 1/66

(54) **Verwendung von alkoxylierten Hydroxycarbonsäureestern zur Lösungsvermittlung von Parfümölen in Wasser**
Use of alkoxylated hydroxy carboxylic acid esters as solubilizers of perfume oils in water
Utilisation des esters d'acide hydroxycarboxylique alkoxylés comme solubilisants des parfums dans l'eau

(30) Priorität: 05.11.2004 DE 102004054036
(43) Veröffentlichungstag der Anmeldung: 24.05.2006
(73) Patentinhaber: Cognis IP Management GmbH, 40589 Düsseldorf (DE)
(72) Erfinder: Albers, Thomas, 40597 Düsseldorf (DE); Behler, Ansgar, 46240 Bottrop (DE)

(56) Entgegenhaltungen:
- EP-A- 0 199 131
- EP-A- 0 209 910
- EP-A- 0 261 351
- EP-A- 0 895 808
- WO-A-94/10970
- WO-A-98/00506
- US-A- 4 170 655
- US-B1- 6 762 157

## Beschreibung

Die vorliegende Erfindung betrifft die Verwendung von alkoxylierten Hydroxycarbonsäureestern als Lösungsvermittler für Parfümöle in Wasser. Derartige Lösungsvermittler können beispielsweise in Reinigungsmitteln eingesetzt werden.

Reinigungsmittel für den institutionellen wie für den privaten Gebrauch enthalten neben den eigentlichen reinigenden Inhaltsstoffen in zunehmendem Maße auch Parfümöle, um den Produkten einen angenehmen Geruch zu vermitteln, der auch möglichst während der Anwendung noch anhalten soll. Man denke hier insbesondere an Produkte für die Reinigung von Toiletten, wie beispielsweise Toilettensteine oder Toilettengele. Es hat sich gezeigt, dass die Verwendung von hohen Anteilen von Parfümölen zu Problemen führt, da diese bei Kontakt mit Wasser nicht immer ausreichend gelöst werden.
Es besteht daher ein Bedarf, derartige Parfümöle, die in der Regel wasserunlöslich sind, durch geeignete Hilfsmittel, die sogenannten Solubilisatoren, in Wasser zu lösen bzw. zu dispergieren. Aus der WO 94/10970 A1 sind für diesen Verwendungszweck Monoalkylcitrate bekannt, deren Alkylgruppen 7 bis 10 Kohlenstoffatome aufweisen müssen. Diese Monoalkylcitrate eigenen sich zur Solubilisierung von Parfümölen in sogenannten Personal Care and Household Products. Allerdings besteht ein ständiger Bedarf, weitere geeignete und verbesserte Solubilisatoren zu finden, wobei insbesondere ein Zusatznutzen der Inhaltsstoffe gewünscht ist. Es wurde gefunden, dass bestimmte Hydroxyarbonsäureesterderivate diese Aufgabe erfüllen können.

WO 98/00506 offenbart Mikroemulsionen für Reinigungsmittel. Die Mikroemulsionen enthalten unter auderem Parfümöle und ein Tensid gemisch 1 - 25% Alkylethoxycitrat können darin enthalten sein.

Gegenstand der vorliegenden Erfindung ist die Verwendung von Estern von Hydroxycarbonsäuren mit alkoxylierten Fettalkoholen als Lösungsvermittler für Parfümöle in Wasser.

Lösungsvermittler sind dabei solche Stoffe, die durch ihre Gegenwart andere, in einem bestimmten Lösungsmittel, vorliegend Wasser, praktisch unlösliche Verbindungen in diesem Lösungsmittel lösen oder emulgierbar machen (Solubilisation).

Alkoxylierte Ester von Hydroxycarbonsäuren sind bereits bekannt und werden beispielsweise in der EP-A2-0 199 131 beschrieben. Dieses Dokument offenbart konkret niedrig ethoxylierte Citronensäurealkylester als wasserlösliche Tenside, insbesondere für den Einsatz in kosmetischen Formulierungen. Dieses Dokument macht keine Angaben darüber, dass die ethoxylierten Citronensäureester eine Eignung zur Solubilisierung von Parfümölen in Wasser aufweisen können. Die Herstellung der erfindungsgemäß verwendeten Estern von Hydroxycarbonsäuren mit alkoxylierten Fettalkoholen ist beispielsweise in der WO 2004/056741-A1 beschrieben. Dazu werden ethoxylierte Fettalkohole mit Citronensäure in einem Rührbehälter mit Wasserabscheider vorgelegt und dann auf Temperaturen bis 160°C erhitzt. Nach Abschluss der Reaktion wird das Gemisch abgekühlt und der Ester durch Destillation gewonnen.

Bei den erfindungsgemäß verwendeten Hydroxycarbonsäuren handelt es sich vorzugsweise um sogenannte Fruchtsäuren. Fruchtsäuren stellen einen Sammelbegriff für vielfach in Früchten (Obst) vorkommende organische Säuren, die neben der Aromabildung und der antimikrobiellen Wirkung noch eine Reihe anderer erwünschter Effekte in Lebensmitteln aufweisen. Die Fruchtsäuren sind erfindungsgemäß vorzugsweise ausgewählt aus der Gruppe Citronensäure, Milchsäure, Apfelsäure, Weinsäure und Gluconsäure. Solche Fruchtsäuren werden heute meist biotechnologisch gewonnen, wie z.B. die Zitronensäure aus Melasse durch Fermentation mit Aspergilllus nigra oder die Gluconsäure durch enzymatische Oxidation von Glucose. Eine bevorzugt Hydroxycarbonsäure im Sinne der vorliegenden technischen Lehre ist die Citronensäure. Ebenfalls bevorzugt ist die Verwendung von Oligo- bzw. Polycarbonsäuren. D.h. es werden solche Carbonsäuren bevorzugt, die mindestens 2 Carboxylsäurefunktionen -COOH im Molekül enthalten. Es können aber auch Hydroxycarbonsäuren mit 3 bis 6 Carbonsäuregruppen im Molekül verwendet werden. Eine Hydroxycarbonsäure im vorliegenden Sinne bedeutet, dass neben mindestens einer Carbonsäuregruppe noch mind. eine freie Hydroxylgruppe im Molekül enthalten sein muss.

Die Hydroxycarbonsäuren werden, wie oben beschrieben, vorzugsweise mit alkoxylierten Fettalkoholen zu den eigentlich wirksamen Esterverbindungen umgesetzt. Die alkoxylierten Fettalkohole folgen vorzugsweise der allgemeinen Formel (I)

R-O-(C₃H₆O)ₘ(C₂H₄-O-)ₙ-H (I)

wobei in der Formel (I) die Indices n und m unabhängig voneinander für Null oder eine Zahl zwischen 1 und 10 stehen können, mit der Maßgabe, dass die Summe aus n und m mindestens 1 sein muss, und der Rest R für einen gesättigten, ungesättigten, verzweigten oder unverzweigten Alkylrest mit 8 bis 22 C-Atomen steht. Es ist bevorzugt, dass solche alkoxylierten Fettalkohole der Formel (I) verwendet werden, in der m den Wert 0 hat und n eine Zahl zwischen 1 uns 10, vorzugsweise zwischen 2 und 8 und insbesondere zwischen 4 und 7 bedeutet. Dabei sind weiterhin solche Fettalkohole der Formel (I) bevorzugt, in denen R in der Formel (I) für gesättigte, unverzweigte Alkylreste mit 8 bis 22, vorzugsweise 12 bis 18 C-Atomen steht. Als Alkoxylate können sowohl ethoxylierte als auch rein propoxylierte Verbindungen eingesetzt werden, wobei es bevorzugt seien kann ethoxylierte und propoxylierte Verbindungen der Formel (I) für die Herstellung der erfindungsgemäßen Ester einzusetzen.
Vorzugsweise werden Verbindungen der Formel (I) ausgewählt in denen m Null ist und n für eine Zahl von 1 bis 10 vorzugsweise 2 bis 8 und insbesondere von 5 bis 7 steht. Sofern rein propoxylierte Verbindungen ausgewählt werden sind solche Verbindungen der Formel (I) bevorzugt in denen n Null ist und m für eine Zahl von 1 bis 6, vorzugsweise 1 bis 4 und insbesondere von 2 bis 4 steht. Im Falle von ethoxylierte und propoxylierten Isomeren der Formel (I) sind Werte für m von 1 bis 2 und für n von 4 bis 8 bevorzugt. Die Abfolge der Alkoxyd-Gruppen in der Formel (I) ist hierbei beliebig. Das heißt, dass sowohl zufällige Abfolgen von Ethylen- und Propylenoxid-Gruppen von der allgemeinen Formel umfasst sind, als auch solche Produkte, die diese Alkoxydgruppen in Blöcken enthalten. Keineswegs ist die Formel (I) dahingehend auszulegen, dass eine Abfolge von Propylenoxiden und anschließend Ethylenoxiden vorliegen müsste. Allerdings sind Verbindungen der Formel (I) bevorzugt, bei denen zunächst ein Block Propylenoxid und anschließend ein Block Ethylenoxid aufeinander folgen.

Bei den erfindungsgemäß verwendeten Estern der Hydroxycarbonsäuren im Sinne handelt es sich um Tenside, die vorzugsweise noch eine freie Carboxylgruppe enthalten. Dementsprechend kann es sich auch um saure Ester oder deren Neutralisationsprodukte handeln. Vorzugsweise liegen die Ester dann in Form von Alkali-, Erdalkali-, Ammonium-, Alkylammonium-, Alkanolammonium- und/oder Glucammoniumsalze vor.

Die Ester selbst leiten sich bevorzugt von ethoxylierten Fettalkoholen der Formel (I) ab, die mit den Hydroxycarbonsäuren umgesetzt werden. Es ist auch möglich, solche Ester erfindungsgemäß zu verwenden, die hergestellt werden indem man Abmischung von alkoxylierten, vorzugsweise ethoxylierten Fettalkoholen der allgemeinen Formel (I) mit Fettalkoholen der allgemeinen Formel

R-OH (II)

mit den Hydroxycarbonsäuren umsetzt. Dabei steht in der Formel (II) R für einen gesättigten, ungesättigten, verzweigten oder unverzweigten Alkylrest mit 8 bis 22 C-Atomen. Bevorzugt ist es dann, dass das Gewichtsverhältnis zwischen den Alkoholen der Formel (II) und den ethoxylierten Alkoholen der Formel (I) im Bereich von 10 : 1 bis 1 : 10 liegt. Als besonders bevorzugt hat es sich erwiesen, die Alkohole der Formel (II) und (I) im Gewichtsverhältnis von 10 : 1 bis 1 : 1 und insbesondere von 9 : 1 bis 1 : 1, vorzugsweise 4 : 1 bis 1 : 1 und dabei insbesondere von 1 : 1 zur Reaktion einzusetzen.

Die Ester gemäß der vorliegenden Erfindung sind vorzugsweise Polyester, sofern mehrere Carboxylfunktionen verestert sind. Typischerweise liegen herstellungsbedingt Gemische der Ester vor, wobei überwiegend Monoester in der Reaktionsmischung enthalten sind. Dabei sind vorzugsweise 75 bis 95 Gew.-% Monoester, 25 bis 5 Gew.-% Diester und in untergeordneten Mengen von 0,01 bis 5 Gew.-% Triester - jeweils bezogen auf die veresterte Komponente - enthalten.

Ein weiterer Gegenstand der vorliegenden Erfindung betrifft Mischungen von isomeren Verbindungen der allgemeinen Formel (III) 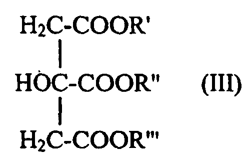 in der R', R", R'" für ein Wasserstoffatom, und/oder einen Alkylrest mit 6 bis 22 C-Atomen und/oder einen ethoxylierten Alkylrest mit 6 bis 22 C-Atomen steht, wobei in den ethoxylierten Alkylresten jeweils zwischen 2 und 20 Teile Ethylenoxid pro Alkylrest enthalten sind, mit den Maßgaben, daß mindestens einer der Reste R', R' bzw. R''' einen solchen ethoxylierten Alkylrest darstellt und die Gesamtzahl an Ethylenoxideinheiten pro Estermolekül auf 20 begrenzt ist. Die Mischungen enthalten Mono- Di- und Triester nebeneinander, wobei Mono- und Diester bevorzugt im Verhältnis von 3 : 1 bis 10 : 1 nebeneinander vorliegen können. Der Anteil an freier Citronensäure kann bei bis zu 20 %, bezogen auf die Mischungen, liegen. Vorzugsweise enthalten die Mischungen aber weniger freie Citronensäure, wobei weniger als 10 Gew.-%%, vorzugsweise weniger als 7 Gew.-% und insbesondere weniger als 5 Gew.-% bevorzugt sind. Im Idealfall sind die Mischungen sogar frei von Zitronensäure.

Die vorliegende technische Lehre betrifft die Verwendung der oben bezeichneten Hydroxycarbonsäurederivaten zur Solubilisierung von Riechstoffen in Wasser. Die Riechstoffe können dabei sowohl bei Raumtemperatur (21 °C) fest als auch flüssig sein, wobei vorzugsweise flüssige Reichstoffe bzw. Parfümöle bevorzugt sind. Dabei werden solche Verbindungen bevorzugt eingesetzt, die in Wasser nur schlecht löslich oder unlöslich sind. Schlechte Löslichkeit bedeutet z.B. dass der Riechstoff sich bei 21 °C nur zu maximal 5 Gew.-%, vorzugsweise maximal 1 Gew.-% bzw. zu maximal 0,1 Gew.-% in Wasser lösen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

Erfindungsgemäß besonders bevorzugt ist die Verwendung der oben beschriebenen alkoxylierten Hydroxycarbonsäureester mit Parfümölen bzw. Riechstoffen auf Basis ungesättigter aliphatischer Aldehyde, vorzugsweise von Octylaldehyd.
Einen ebenso bevorzugten Riechstoff stellt das Cumol dar. Des Weiteren sind Orangenöl und/oder Zitronenterpen bevorzugte Riechstoffe im Sinne der vorliegenden Erfindung.
Die Parfümöle können alleine oder als Mischungen untereinander mittels der oben beschriebenen Hydroxycarbonsäure in Wasser solubilisiert werden. Es ist auch möglich, die Riechstoffe zunächst in einem nichtwässerigen Lösungsmittel, z.B. einem Alkohol, wie Ethanol oder Propanol zu lösen und danach den gelösten Riechstoff mit den erfindungsgemäßen Hydroxycarbonsäuren zu solubilisieren.

Die erfindungsgemäß eingesetzten Ester von Hydroxycarbonsäuren mit alkoxylierten Fettalkoholen eigenen sich als Lösungsvermittler für Parfümöle in Wasser. Überraschenderweise weisen sie allerdings noch einen Zusatznutzen auf, nämlich den Umstand, dass sie eine komplexierende Wirkung insbesondere auf Calciumionen ausüben. Somit vereinen die erfindungsgemäß verwendeten alkoxylierten Hydroxycarbonsäuren zwei Produkteigenschaften in einer Substanz: Durch die erfindungsgemäße Verwendung wird es überflüssig, neben den Solubilisatoren noch weitere zusätzliche Komplexierungsmittel, wie es beispielsweise Phosphonate, freie Fruchtsäuren, starke Säuren oder z.B. EDTA sein können einzusetzen.
Ein weiterer erfindungsgemäßer Vorteil liegt darin, dass bei der Verwendung im Sinne der vorliegenden Erfindung der Rückstand in der Reinigungsflotte dispergiert wird. Im Gegenteil dazu treten bei gebräuchlichem Komplexierungsmitteln solche Rückstände auf, die auf der Flüssigkeitsoberfläche aufschwimmen. Durch die erfindungsgemäße Verwendung der alkoxylierten Hydroxycarbonsäure kann man daher solche Kalkrückstände besser mit der Reinigungslösung entfernen.

Die alkoxylierten Ester der Hydroxycarbonsäuren im Sinne der vorliegenden Erfindung können alleine, aber vorzugsweise in Kombination mit anderen Inhaltsstoffen z.B. und vorzugsweise in Reinigungsmittel verwendet werden. Andere Inhaltsstoffe können übliche dem Fachmann für derartige Anwendungen bekannte Mittel sein, vorzugsweise weitere Tenside aus der Gruppe der nichtionischen, anionischen, kationischen und/oder amphoteren Tenside. Nichtionische Tenside im Rahmen der vorliegenden Erfindung können alkoxylierte Alkohole, wie Polyglycolether, Fettalkoholpolyglycolether, Alkylphenolpolyglycolether, endruppenverschlossene Polyglycolether, Mischether, Hydroxymischether, alkoxylierte Carbonsäureester, Aminoxide und Alkylpolyglykoside sein. Ebenfalls verwendbar sind Ethylenoxid-Propylenoxid-Blockpolymere und Fettsäurealkanolamide und Fettsäurepolyglycolether. Besonders bevorzugt sind in den erfindungsgemäßen Mitteln als nichtionische Tenside Alkylpolyglykoside alleine oder in Mischung mit weiteren nichtionischen Tensiden enthalten.
Alkylpolyglykoside stellen bekannte nichtionische Tenside dar, die der Formel RO-[G]p folgen, in der R für einen Alkyl- und/oder Alkenylrest mit 6 bis 22 Kohlenstoffatomen, G für einen Zuckerrest mit 5 oder 6 Kohlenstoffatomen und p für eine Zahl zwischen 1 und 10 steht. Sie können nach den einschlägigen Verfahren der präparativen organischen Chemie erhalten werden. Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligoglucoside. Die Indexzahl p in der allgemeinen Formel (I) gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C8-C10 (DP = 1 bis 3), die als Vorlauf bei der destillativen Auftrennung von technischem C8-C18-Kokosfettalkohol anfallen und mit einem Anteil von weniger als 6 Gew.-% C12-Alkohol verunreinigt sein können sowie Alkyloligoglucoside auf Basis technischer C9/11-Oxoalkohole (DP = 1 bis 3). Der Alkyl- bzw. Alkenylrest R kann sich ferner auch von primären Alkoholen mit 12 bis 22, vorzugsweise 12 bis 14 Kohlenstoffatomen ableiten. Typische Beispiele sind Laurylalkohol, Myristylalkohol; Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C12/14- Kokosalkohol mit einem DP von 1 bis 3.
Die erfindungsgemäßen Mittel können die nichtionischen Tenside, vorzugsweise die Alkylpolyglykoside alleine oder in Mischung mit den Aminoxiden, als alleiniges Tensid enthalten. Es ist aber auch möglich, daß die erfindungsgemäßen Mittel zusätzlich anionische und vorzugsweise kationische und/oder amphotere bzw. zwitterionische Tenside enthalten. In geringen Mengen können anionische Tenside, beispielsweise aliphatische Sulfate wie Fettalkoholsulfate, Fettalkoholethersulfate, Fettsäurepolyglykolestersulfate, Dialkylethersulfate, Monoglyceridsulfate und aliphatische Sulfonate wie Alkansulfonate, Olefinsulfonate, Ethersulfonate, n-Alkylethersulfonate, Estersulfonate, und Lingninsulfonate enthalten sein.
Als anionische Tenside werden bevorzugt Fettalkoholsulfate, Fettalkoholethersulfate und/oder Fettsäurepolyglykolestersulfate. Da die anionischen Tenside jedoch mit den kationischen Polymeren als Verdickungsmittel in Wechselwirkung treten, kann es insbesondere bei starken anionischen Tensiden zu Ausfällungen kommen, vor allen Dingen auch dann, wenn die Menge an anionischen Tensiden über 10 Gew.-% ― bezogen auf Gesamttensidmenge ― beträgt.
Um klare Mittel zu gewährleisten, ist es im Sinne der vorliegenden Erfindung besonders bevorzugt, wenn die erfindungsgemäßen Mittel wenig, d.h. weniger als 2,5 Gew.-% vorzugsweise weniger als 1,5 Gew.-% und vorzugsweise überhaupt keine anionischen Tenside enthalten. Alkoxylierte Fettalkoholhydroxycarbonsäureester im Sinne der vorliegenden Erfindung werden daher vorzugsweise in solchen Mitteln verwendet die wenig oder keine anionischen Tenside enthalten.
Als kationische Tenside können die erfindungsgemäßen Mittel quartäre Ammoniumverbindungen und Esterquats, insbesondere quaternierte Fettsäuretrialkanolaminester-Salze enthalten. Besonders bevorzugt kann die Mitverwendung von Aminoxiden und/oder von Alkylglycosiden sein.

Auch Buildersubstanzen und/oder andere Inhaltsstoffe, beispielsweise Desinfektionsmittel, Biozide, Farbstoffe, Verdicker und vorzugsweise polymere Verdicker, pH-Regulantien, nichtwässerige Lösungsmittel, wie vorzugsweise Ethanol, Propanol oder Ethylenglykol und dessen Derivate können enthalten sein. Sofern schaumförmige Mittel betroffen sind, können noch Treibmittel enthalten sein. Auch Konservierungsmittel können mitverwendet werden. Die Reinigungsmittel können auch kalklösende Mittel enthalten, vorzugsweise freie Zitronensäure. Aufgrund der oben geschilderten Eigenschaften der erfindungsgemäß verwendeten Hydroxycarbonsäurestern ist die Mitverwendung weiterer Komplexierungsmittel, insbesondere für Calcium-Ionen nicht bevorzugt und vorzugsweise ausgeschlossen. Bevorzugte Anwendungsgebiete im Sinne der vorliegenden technischen Lehre finden sich bei Reinigungsmitteln für harte Oberflächen, vorzugsweise bei Toilettenreinigern, wobei die Hydroxycarbonsäuren vorzugsweise in flüssigen oder gelförmigen Reinigern zum Einsatz kommen. Auch eine Verwendung in schaumförmigen Mitteln ist bevorzugt. Weiterhin bevorzugt ist die Verwendung in sog. Air-Freshener, d.h. wässerigen Zubereitungen zur Parfümierung von Luft. Es ist aber auch möglich, Parfümöle und das Solubilisierungsmittel getrennt voneinander zu lagern und beispielsweise in Form einer geeigneten Dosiervorrichtung erst kurz vor der eigentlichen Anwendung zu vermischen.

Wenn die erfindungsgemäßen Ester andere Inhaltsstoffe zu einem Endprodukt formulieren ist darauf zu achten, dass vorzugsweise der pH-Wert dieser Mittel im Bereich von 4 bis 7, vorzugsweise von 4,5 bis 6,5 liegt. Es ist weiterhin bevorzugt, die ethoxylierten bzw. alkoxylierten Ester im Sinne der vorliegenden Erfindung zusammen mit Parfümölen in einem Gewichtsverhältnis einzusetzen, das vorzugsweise zwischen 10 : 1 bis 1 : 1 liegen sollte. Im Maximum können 60 Gew.-% an Parfümölen bezogen auf die eingesetzte Menge der erfindungsgemäßen Ester verwendet werden.

### Beispiele

### I. Herstellung der Ester

1.
   Herstellung eines erfindungsgemäßen Esters aus Citronensäure mit einem C₁₂-C₁₈ Fettalkohol + 7 EO: 28,05 kg (0,146 kmol) wasserfreie Citronensäure wurde mit 75,16 kg (0,146 Kmol) Dehydol® LT 7 ein Handelsprodukt der Cognis Deutschland GmbH & Co. KG, eine mit 7 Mol Ethylenoxid ethoxylierte Fettalkoholmischung der folgenden Kettenverteilung (in Gew.-%) : < C12: 0-3%; C12: 48-58%; C14: 18-24%; C16: 8-12%; C18: 11-15 %; > C18: 0-1 % in einem Rührbehälter unter Stickstoff auf 160 °C aufgeheizt und solange bei dieser Temperatur gerührt bis die theoretische Menge an Wasser freigesetzt worden war (5,5 Stunden). Man erhielt ein hellgelbes, klares und flüssiges Produkt mit folgenden Kennzahlen: Verseifungszahl (DIN): 222, Säurezahl (DIN): 132, Freie Citronensäure: 2,8 Gew.-%.
2.
   Analog zu der Herstellung unter 1. wurde ein weiterer Zitronensäureester hergestellt. Statt des Fettalkohols Dehydol® LT 7 wurde eine äquimolare Menge einer Fettalkoholmischung auf Basis von C10/C16 gesättigten Fettalkoholen, die mit 1,2 Teilen Propylenoxid (PO) und 6,4 Teilen Ethylenoxid (EO) pro Teil Fettalkohol umgesetzt worden sind. Es wurde ein hellgelbes, klares und flüssiges Produkt mit den folgenden Kennzahlen erhalten: Säurezahl: 166, Verseifungszahl: 243.

### II. Anwendungstechnische Prüfungen

1.
   Um die erfindungsgemäße Wirkung zu prüfen, wurden eine 10 Gew.-%ige wässerige
   Lösung von verschiedenen Tensiden hergestellt. Zur Herstellung wurde zunächst das Tensid mit dem Parfümöl gemischt und anschließend Wasser zugegeben.. Je höher der Anteil an Parfümöl desto besser ist die Solubilisierungswirkung des Tensids.
   In der folgenden Tabelle 1 steht V für Produkte des Standes der Technik und E für erfindungsgemäße, alkoxylierte Ester.
   Die erfindungsgemäßen Beispiele E1 und E2 benutzen einen Citronensäureester gemäß der obigen Beschreibung I.1 bzw. I.2.

**Tabelle 1**

| **Nr.** | **Tensid** | **Parfümöl** | **Menge bis zur Trübung in Gew.-%** |
|---|---|---|---|
| **V1a** | 2-Hydroxy-C12-(PO)₁-(EO)₉-Glykolether | Orangenöl | 4 |
| **V1b** | Wie bei V1a | Zitronenterpen | 4 |
| **V1c** | Wie bei V1a | Octylaldehyd | 2 |
| **V2a** | gehärtetes Rizinusöl mit 40 Teilen Ethylenoxid (EO) | Orangenöl | 4 |
| **V2c** | Wie bei V2a | Octylaldehyd | 4 |
| **V3a** | Mischung aus C12-C18 Fettalkohol + 7 EO, C8-C10 APG, Natriumlaurylethersulfat + 2 EO entsprechend der Offenbarung der EP 1 441 024 | Orangenöl | 3 |
| **V3b** | wie bei V3a | Zitronenterpen | 3 |
| **E1a** | Ester aus Laurylcitrat + C12-C18 mit Fettalkohol + 7 EO | Orangenöl | 6 |
| **E1b** | Wie bei E1a | Zitronenterpen | 5 |
| **E1c** | Wie bei E1a | Octylaldehyd | 9,5 |
| **E2a** | Ester aus Beispiel I.2 | Orangenöl | 9,5 |
| **E2b** | Wie bei E2a | Zitronenterpen | 9,5 |
| **E2c** | Wie bei E2a | Octylaldehyd | 7,5 |

Die erfindungsgemäßen Beispiele E1 und E2 zeigen jeweils einen deutlich höheren Anteil an gelöstem Parfümöl als die Solubilisatoren V1 bis V3 des Standes der Technik.

## Patentansprüche

1. Verwendung von Estern von Hydroxycarbonsäuren mit alkoxylierten Fettalkoholen als Lösungsvermittler für Riechstoffe in Wasser.

2. Verwendung nach Anspruch 1, **dadurch gekennzeichnet, dass** bei Raumtemperatur flüssige Parfümöle als Riechstoffe ausgewählt sind.

3. Verwendung nach den Ansprüchen 1 bis 2, **dadurch gekennzeichnet, dass** die Hydroxycarbonsäuren ausgewählt sind aus der Gruppe Citronensäure, Milchsäure, Äpfelsäure, Weinsäure, Gluconsäure.

4. Verwendung nach den Ansprüchen 1 bis 3, **dadurch gekennzeichnet, dass** als Hydroxycarbonsäure Citronensäure ausgewählt ist.

5. Verwendung nach den Ansprüchen 1 bis 4, **dadurch gekennzeichnet, dass** die alkoxylierten Fettalkohole der allgemeinen Formel (I)
R-O-(C₃H₆O)ₘ (C₂H₄-O-)ₙ -H (I)
folgen, wobei in der Formel (I) die Indices n und m unabhängig voneinander für Null oder eine Zahl zwischen 1 und 10 stehen können, mit der Maßgabe, dass die Summe aus n und m mindestens 1 sein muss, und der Rest R für einen gesättigten, ungesättigten, verzweigten oder unverzweigten Alkylrest mit 8 bis 22 C-Atomen steht.

6. Verwendung nach den Ansprüchen 1 bis 5, **dadurch gekennzeichnet, dass** die alkoxylierten Fettalkohole der Formel (I) folgen, in der m den Wert Null hat und n eine Zahl zwischen 1 und 10, vorzugsweise zwischen 2 und 8 und insbesondere zwischen 4 und 7 bedeutet.

7. Verwendung nach den Ansprüchen 1 bis 6, **dadurch gekennzeichnet, dass** R in der Formel (I) für gesättigte, unverzweigte Alkylreste mit 8 bis 22, vorzugsweise 12 bis 18 C-Atomen steht.

8. Verwendung nach einem der Ansprüche 1 bis 7, **dadurch gekennzeichnet, dass** das Parfümöl ausgewählt ist aus der Gruppe Orangenöl und/oder Zitronenterpen.

9. Verwendung nach einem der Ansprüche 1 bis 8, **dadurch gekennzeichnet, dass** als Parfümöl Octylaldehyd ausgewählt ist.

10. Verwendung nach einem der Ansprüche 1 bis 9, **dadurch gekennzeichnet, dass** als Parfümöl Cumol ausgewählt ist.

11. Verwendung nach einem der Ansprüche 1 bis 10, **dadurch gekennzeichnet, dass** alkoxylierte Partialester von Hydroxycarbonsäuren mit mindestens zwei Carboxylfunktionen verwendetet werden.

12. Verwendung nach einem der Ansprüche 1 bis 11, **dadurch gekennzeichnet, dass** Citronensäureestermischungen von ethoxylierten Alkoholen der allgemeinen Formel (I) in der R für einen linearen Alkylrest abgeleitet von einer Fettalkoholmischung enthaltend 45 - 75 Gew.-% C12-, 15 bis 35 Gew.-% C14-, 0 - 15 Gew.-% C 16- und 0 bis 20 Gew.-% C 18- Alkohol und n für Zahlen von 5 bis 9 steht, mit der Maßgabe, dass in den Citronensäureestermischungen das Gewichtsverhältnis von Monoester : Diester im Bereich von 3 : 1 bis 10 : 1 liegt.

13. Verwendung nach einem der Ansprüche 1 bis 12, **dadurch gekennzeichnet, dass** die alkoxylierte Fettalkoholhydroxycarbonsäureester als Lösungsvermittler in Reinigungsmitteln eingesetzt werden.

14. Verwendung nach einem der Ansprüche 1 bis 13, **dadurch gekennzeichnet, dass** die Hydroxycarbonsäuren nicht zusammen mit anionischen Tensiden in einem Mittel bzw. einer Formulierung verwendet werden.

## Claims

1. Use of esters of hydroxycarboxylic acids with alkoxylated fatty alcohols as solubilizers for perfumes in water.

2. Use claimed in claim 1, **characterized in that** perfume oils liquid at room temperature are selected as the perfumes.

3. Use claimed in claims 1 and 2, **characterized in that** the hydroxycarboxylic acids are selected from the group consisting of citric acid, lactic acid, malic acid, tartaric acid, gluconic acid.

4. Use claimed in claims 1 to 3, **characterized in that** citric acid is selected as the hydroxycarboxylic acid.

5. Use claimed in claims 1 to 4, **characterized in that** the alkoxylated fatty alcohols correspond to general formula (I):
R-O-(C₃H₆O)ₘ(C₂H₄-O-)ₙ-H (I)
in which the indices n and m independently of one another stand for 0 and for a number of 1 to 10, with the proviso that the sum of n and m must be at least 1, and the substituent R is a saturated, unsaturated, branched or unbranched C₈₋₂₂ alkyl group.

6. Use claimed in claims 1 to 5, **characterized in that** the alkoxylated fatty alcohols correspond to formula (I) where m = 0 and n is a number of 1 to 10, preferably 2 to 8 and more particularly 4 to 7.

7. Use claimed in claims 1 to 6, **characterized in that** R in formula (I) represents saturated, unbranched alkyl groups containing 8 to 22 and preferably 12 to 18 carbon atoms.

8. Use claimed in any of claims 1 to 7, **characterized in that** the perfume oil is selected from the group consisting of orange oil and/or lemon oil terpene.

9. Use claimed in any of claims 1 to 8, **characterized in that** octyl aldehyde is selected as the perfume oil.

10. Use claimed in any of claims 1 to 9, **characterized in that** cumene is selected as the perfume oil.

11. Use claimed in any of claims 1 to 10, **characterized in that** alkoxylated partial esters of hydroxycarboxylic acids containing at least two carboxyl functions are used.

12. Use claimed in any of claims 1 to 11, **characterized in that** citric acid ester mixtures of ethoxylated alcohols of general formula (I), where R is a linear alkyl group derived from a fatty alcohol mixture containing 45 to 75% by weight C12 alcohol, 15 to 35% by weight C14 alcohol, 0 to 15% by weight C16 alcohol and 0 to 20% by weight C18 alcohol and n is a number of 5 to 9, with the proviso that the ratio by weight of monoester to diester in the citric acid ester mixtures is in the range from 3:1 to 10:1, are used.

13. Use claimed in any of claims 1 to 12, **characterized in that** the alkoxylated fatty alcohol hydroxycarboxylic acid esters are used as solubilizers in cleaning compositions.

14. Use claimed in any of claims 1 to 13, **characterized in that** the hydroxycarboxylic acids are not used together with anionic surfactants in a composition or formulation.

## Revendications

1. Utilisation d'esters d'acides hydroxycarboxyliques avec des alcools gras alcoxylés, en tant que tiers-solvants pour parfums dans de l'eau.

2. Utilisation selon la revendication 1, **caractérisée en ce qu'**on choisit comme parfums des huiles essentielles liquides à la température ambiante.

3. Utilisation selon les revendications 1 et 2, **caractérisée en ce que** les acides hydroxycarboxyliques sont choisis dans le groupe constitué par l'acide citrique, l'acide lactique, l'acide malique, l'acide d-tartrique, l'acide gluconique.

4. Utilisation selon les revendications 1 à 3, **caractérisée en ce qu'**on choisit comme acide hydroxycarboxylique l'acide citrique.

5. Utilisation selon les revendications 1 à 4, **caractérisée en ce que** les alcools gras alcoxylés correspondent à la formule générale (I)
R-O-(C₃H₆O)ₘ(C₂H₄-O-)ₙ-H (I)
dans la formule (I) les indices n et m pouvant représenter, indépendamment l'un de l'autre, zéro ou un nombre compris entre 1 et 10, étant entendu que la somme de n et m doit au moins être égale à 1, et le radical R représente un radical alkyle saturé, insaturé, ramifié ou non ramifié, ayant de 8 à 22 atomes de carbone.

6. Utilisation selon les revendications 1 à 5, **caractérisée en ce que** les alcools gras alcoxylés correspondent à la formule (I), dans laquelle m a la valeur zéro et n représente un nombre compris entre 1 et 10, de préférence entre 2 et 8 et en particulier entre 4 et 7.

7. Utilisation selon les revendications 1 à 6, **caractérisée en ce que** R dans la formule (I) représente des radicaux alkyle saturés, non ramifiés, ayant de 8 à 22, de préférence de 12 à 18 atomes de carbone.

8. Utilisation selon l'une quelconque des revendications 1 à 7, **caractérisée en ce que** l'huile essentielle est choisie dans le groupe constitué par l'huile d'orange et/ou le terpène de citron.

9. Utilisation selon l'une quelconque des revendications 1 à 8, **caractérisée en ce qu'**on choisit comme huile essentielle l'octylaldéhyde.

10. Utilisation selon l'une quelconque des revendications 1 à 9, **caractérisée en ce qu'**on choisit comme huile essentielle le cumène.

11. Utilisation selon l'une quelconque des revendications 1 à 10, **caractérisée en ce qu'**on utilise des esters partiels alcoxylés d'acides hydroxycarboxyliques comportant au moins deux fonctions carboxy.

12. Utilisation selon l'une quelconque des revendications 1 à 11, **caractérisée en ce qu'**on utilise des mélanges d'esters d'acide citrique avec des alcools éthoxylés de formule générale (I) dans laquelle R représente un radical alkyle linéaire dérivé d'un mélange d'alcools gras contenant 45 - 75 % en poids d'alcool en C₁₂, 15 à 35 % en poids d'alcool en C₁₄, 0 - 15 % en poids d'alcool en C₁₆ et 0 à 20 % en poids d'alcool en C₁₈ et n représente des nombres valant de 5 à 9, étant entendu que dans les mélanges d'esters d'acide citrique le rapport pondéral monoesters:diesters se situe dans la plage de 3:1 à 10:1.

13. Utilisation selon l'une quelconque des revendications 1 à 12, **caractérisée en ce que** les esters d'acides hydroxycarboxyliques avec des alcools gras alcoxylés sont utilisés comme tiers-solvants dans des produits de nettoyage.

14. Utilisation selon l'une quelconque des revendications 1 à 13, **caractérisée en ce que** les acides hydroxycarboxyliques ne sont pas utilisés conjointement avec des tensioactifs anioniques dans un produit ou une composition.
